(19)

Europäisches Patentamt
European Patent Office
Office européen des brevets

(11)  **EP 4 732 757 A1**

(12)  **EUROPEAN PATENT APPLICATION**

(43) Date of publication:
**29.04.2026  Bulletin 2026/18**

(21) Application number: **24208564.5**

(22) Date of filing: **24.10.2024**

(51) International Patent Classification (IPC):
**A61B 5/00** (2006.01)      **H03M 7/30** (2006.01)

(52) Cooperative Patent Classification (CPC):
**H03M 7/607; A61B 5/7232; A61B 5/726;
H03M 7/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
- **JELFS, Sam Martin**
  **Eindhoven (NL)**
- **OOMEN, Arnoldus Werner Johannes**
  **Eindhoven (NL)**
- **PERRONE, Antonio Luigi**
  **Eindhoven (NL)**
- **DE BONT, Fransiscus Marinus Jozephus**
  **Eindhoven (NL)**

(74) Representative: **Philips Intellectual Property &
Standards
High Tech Campus 52
5656 AG Eindhoven (NL)**

(54)  **GENERATION AND PROCESSING OF DATA SIGNAL COMPRISING DATA FOR A BIOMEDICAL WAVEFORM SIGNAL**

(57)  An apparatus is arranged to generate a data signal comprising compressed data for a biomedical waveform signal and an indication of a property of the compression process used to generate the compressed data from the biomedical waveform signal.

A second apparatus includes a receiver (201) receiving the data signal. A decompressor (303) generates a reconstructed biomedical waveform signal by decompression of the compressed data. Further, the second apparatus comprises an adapter (307) which adapts the decompression in dependence on the indication of the property of the compression. The approach may provide a highly efficient and flexible compression of biomedical waveform signals.

**FIG. 3**

**Description**

FIELD OF THE INVENTION

The invention relates to generation and processing of data signal comprising data for a biomedical waveform signal, and in particular, but not exclusively, of compression and/or decompression of biomedical waveform signal(s) such as an electrocardiography (ECG), electroencephalography (EEG), electromyography (EMG), or photoplethysmography (PPG) signal.

BACKGROUND OF THE INVENTION

The capturing, storing, distributing, and processing of biomedical data has become increasingly important in recent decades as recording such signals has progressed from being performed almost exclusively for short term measurements in dedicated clinical environments, such as hospitals, to becoming increasingly prevalent in the personal space with biomedical measurements often being made continuously over very long periods. For example, the cardiology field has evolved from mainly considering short heart rate waveforms collected in a clinician's office to frequently using chest-worn outpatient devices that are routinely worn for multiple days, and which collect continuous data for long durations.

Consequently, the requirements for data storage, distribution, and processing have grown exponentially. Accordingly, there is a desire to effectively represent captured biomedical waveform signals to facilitate and reduce the requirements for storing, distributing, and/or processing the signals. However, whereas various proprietary approaches have been proposed for encoding or compressing biomedical waveform signals used by various manufacturers, current approaches tend to be suboptimal and tend to not provide ideal performance. Further, current approaches tend to be complex and/or targeted on specific biomedical waveform signals, with poor interoperability to related usages, resulting in specific requirements and limited application.

Hence, an improved approach would be advantageous. In particular, an approach allowing increased flexibility, improved performance, increased quality, reduced complexity, reduced data rate, increased accuracy/reduced distortion of encoding/decoding/compressing/decompressing operations, reduced storage requirements, reduced bandwidth/data rate for biomedical waveform signals, reduced computational load, facilitated implementation, and/or improved performance would be advantageous.

SUMMARY OF THE INVENTION

Accordingly, the invention seeks to preferably mitigate, alleviate or eliminate one or more of the above mentioned disadvantages singly or in any combination.

According to an aspect of the invention, there is provided an apparatus comprising: a receiver arranged to receive a data signal comprising compressed data for a biomedical waveform signal; a decompressor arranged to generate a reconstructed biomedical waveform signal by decompression of the compressed data; wherein the data signal comprises an indication of a property of a compression process used to generate the compressed data from the biomedical waveform signal; and an adapter arranged to adapt the decompression in dependence on the indication of the property of the compression.

The invention may allow advantageous (compression/)decompression of a biomedical waveform signal and may support a more efficient and/or accurate compression/decompression operation. The invention may allow a reduced bandwidth/data rate for compressed data representing a biomedical waveform signal. In many embodiments, the storage requirements and/or required communication bandwidth may be reduced substantially. The approach may allow efficient and practical implementation and/or may reduce complexity and processing/computational requirements.

The biomedical waveform signal may specifically be an electrocardiography (ECG), electroencephalography (EEG), electromyography (EMG), or photoplethysmography (PPG) signal. The biomedical waveform signal may also be an audible signal originating from a measurement on a test subject, such as a heartbeat signal, a breathing signal, or a voice signal, acquired by e.g. an electronic stethoscope or another electronic capturing device suitable for capturing such signals.

According to an optional feature of the invention, the indication of the property of the compression process is indicative of a compression algorithm used to generate the compressed data; and the adapter is arranged to select a decompression algorithm for the decompression from a set of decompression algorithms in dependence on the indication of the property of the compression process.

This may provide a particularly efficient and high performance operation in many scenarios, and may typically result in an improved reconstructed biomedical waveform signal and/or a reduced data size/rate for the compressed biomedical waveform signal.

According to an optional feature of the invention, the set of decompression algorithms includes at least one lossless decompression algorithm and at least one lossy decompression algorithm.

According to an optional feature of the invention, the compressed data includes wavelet transform coefficient data, and the decompression includes applying a wavelet decompression algorithm to the wavelet transform coefficient data.

This may provide a particularly efficient and high performance operation in many scenarios, and may typically result in an improved reconstructed biomedical waveform signal and/or a reduced data size/rate for the compressed biomedical waveform signal.

According to an optional feature of the invention, the indication of the property of the compression process is one of a plurality of indications of the property of the compression process each of which is linked with a time interval, and the adapter is arranged to in each time interval adapt the decompression in dependence on the indication of the property of the compression linked with the time interval.

This may provide a particularly efficient and high performance operation in many scenarios, and may typically result in an improved reconstructed biomedical waveform signal and/or a reduced data size/rate for the compressed biomedical waveform signal. It may in particular allow improved compression/encoding for biomedical waveform signals with varying properties.

According to an optional feature of the invention, the compression employs a wavelet transform, and the indication of the property of the compression process comprises an indication of a mother wavelet for the wavelet transform.

This may provide a particularly efficient and high performance operation in many scenarios, and may typically result in an improved reconstructed biomedical waveform signal and/or a reduced data size/rate for the compressed biomedical waveform signal.

In some embodiments, the indication of the property of the compression process comprises an indication of a father wavelet for the wavelet transform. This may in many, but not all, embodiments be combined with the indication of the property of the compression process being indicative of a mother wavelet.

According to an optional feature of the invention, the compression employs a wavelet transform, and the indication of the property of the compression process comprises an indication of a property of a composition tree for the wavelet transform.

This may be particularly advantageous in many embodiments and scenarios.

According to an optional feature of the invention, the data signal comprises data for at least one further signal and a grouping indication for indicating that the biomedical waveform signal and the at least one further signal belong to a common group; and the decompressor is arranged to generate the reconstructed biomedical waveform signal by generating the reconstructed biomedical waveform signal in dependence on the at least one further signal when the grouping indicator indicates that that the biomedical waveform signal and the at least one further signal belong to the common group.

This may be particularly advantageous in many embodiments and scenarios.

According to an optional feature of the invention, the data signal further comprises a timing synchronization indicator indicative of a timing offset between the biomedical waveform signal and the at least one further signal.

This may be particularly advantageous in many embodiments and scenarios.

According to an optional feature of the invention, the data signal comprises compressed data for a plurality of biomedical waveform signals and a type indicator indicating a biomedical waveform signal type for at least some of the biomedical waveform signals.

According to an optional feature of the invention, the data signal further comprises an indication of an average level of at least part of the biomedical waveform signal, and the decompression is arranged to generate the reconstructed biomedical waveform signal in dependence on the average level.

This may be particularly advantageous in many embodiments and scenarios.

According to an optional feature of the invention, the compression property indication is indicative of segments of the biomedical waveform signal for which no data is included in the compressed data.

This may be particularly advantageous in many embodiments and scenarios.

According to an optional feature of the invention, the data signal comprises at least one event indication for the biomedical waveform signal, the event indication being indicative of a timing of an event, and the adapter is arranged to adapt the decompression in dependence on the event indication.

This may be particularly advantageous in many embodiments and scenarios.

According to an optional feature of the invention, the data signal further comprises at least one indication of a first time interval, and the decompressor is arranged to generate at least a component of the biomedical waveform signal for the first time interval independently of the compressed data.

This may be particularly advantageous in many embodiments and scenarios.

According to an aspect of the invention, there is provided an apparatus for generating a data signal representing a biomedical waveform signal, the apparatus comprising: a receiver arranged to receive the biomedical waveform signal; a compressor arranged to perform a compression process to generate compressed data from/representing the biomedical waveform signal; and a data signal generator arranged to generate the data signal to comprise the compressed data and an indication of a property of the compression process used to generate the compressed data from the biomedical waveform signal.

According to an aspect of the invention, there is provided a data signal comprising: compressed data for a biomedical waveform signal; and an indication of a property of a compression process used to generate the compressed data from the biomedical waveform signal.

In some embodiments there is provided a(computer readable ) an electromagnetic carrier wave embodying/carrying such a data signal.

In some embodiments, the data signal defined above is embodied on a medium or as an electromagnetic carrier wave.

According to an aspect of the invention, there is provided a method of reconstructing a biomedical waveform signal, the method comprising: receiving a data signal comprising compressed data for the biomedical waveform signal; generating a reconstructed biomedical waveform signal by decompression of the compressed data; wherein the data signal comprises an indication of a property of a compression process used to generate the compressed data from the biomedical waveform signal; and the method comprises adapting the decompression in dependence on the indication of the property of the compression.

According to an aspect of the invention, there is provided a method of generating a data signal representing a biomedical waveform signal, the method comprising: receiving the biomedical waveform signal; performing a compression process to generate compressed data from/representing the biomedical waveform signal; and generating the data signal to comprise the compressed data and an indication of a property of the compression process used to generate the compressed data from the biomedical waveform signal.

These and other aspects, features and advantages of the invention will be apparent from and elucidated with reference to the embodiment(s) described hereinafter.

BRIEF DESCRIPTION OF THE DRAWINGS

Embodiments of the invention will be described, by way of example only, with reference to the drawings, in which

FIG. 1 illustrates some elements of an example of a system for processing biomedical waveform signals;
FIG. 2 illustrates some elements of an example of an apparatus for generating a data signal comprising compressed data representing a biomedical waveform signal in accordance with some embodiments of the invention;
FIG. 3 illustrates some elements of an example of an apparatus for generating a reconstructed biomedical waveform signal from a data signal comprising compressed data representing the biomedical waveform signal in accordance with some embodiments of the invention;
FIG. 4 illustrates an example of a wavelet transform filter banks structure;
FIG. 5 illustrates an example of a frequency span for different levels of a wavelet transform structure;
FIG. 6 illustrates an example of a time-frequency tiling for a wavelet transform structure;
FIG. 7 illustrates some elements of an example of a system for processing biomedical waveform signals;
FIG. 8 illustrates some elements of a possible arrangement of a processor for implementing elements of a compression/decompression apparatus in accordance with some embodiments of the invention.

DETAILED DESCRIPTION OF SOME EMBODIMENTS OF THE INVENTION

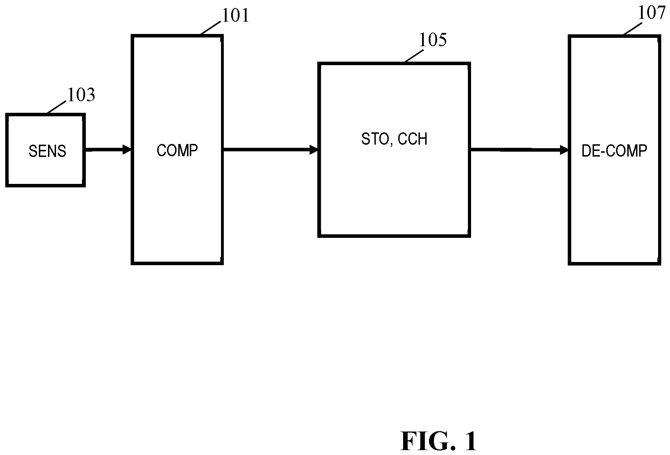

FIG. 1 illustrates an example of a system for generating a data signal comprising compressed data representing a biomedical waveform signal and for reconstructing the biomedical waveform signal from the compressed data.

The system comprises a compression circuit or device 101 which is arranged to receive a biomedical waveform signal from a suitable biomedical sensor.

In many embodiments, the biomedical waveform signal may be an electrocardiography (ECG) signal which specifically may be provided from a sensor comprising a plurality of electrodes that are positioned on the subjects' body to pick up electrical signals relating to the heart operation.

In some embodiments, the biomedical waveform signal may be an electroencephalography (EEG) signal which specifically may be provided from a sensor comprising a number of electrodes that are positioned on the subjects' head to pick up electrical signals relating to brain activity.

In some embodiments, the biomedical waveform signal may be an electromyography (EMG) signal which specifically may be provided from a sensor comprising a plurality of electrodes that are positioned on the subjects' body to pick up electrical signals relating to muscle activation of the person.

In some embodiments, the biomedical waveform signal may be a photoplethysmography (PPG) signal which specifically may be provided from a sensor comprising a photodetector arranged to measure the intensity of reflected light from tissue and skin of a person. Such a detection may, for e.g., provide a measurement of blood volume/flow changes or heart rate.

The biomedical waveform signal is specifically a time domain signal with a time domain waveform that reflects the changes in the signal from the sensor as a function of time.

The compression device 101 proceeds to process the biomedical waveform signal to generate a data signal which includes compressed data representing the biomedical waveform signal.

In many embodiments, the compression device 101 may receive a plurality of biomedical waveform signals which may each be encoded/compressed as described, i.e., compressed data may be generated representing each of the biomedical waveform signals.

The data signal may then be provided to an intermediate circuit/function 105 from which it is provided to a decompressor circuit or device 107 which is arranged to generate a reconstructed biomedical waveform signal from the received data signal, and specifically from the compressed data representing the biomedical waveform signal.

The intermediate circuit/function 105 may for example be a distribution or communication medium/channel/network. For example, the data signal may be communicated from the compression device 101 to the decompressor device 107 via the Internet or another communication network. As another example, the intermediate circuit/function 105 may be a store or memory. For example, in some embodiments, the compression device 101 and the decompressor device 107 may be implemented as part of the same device with the biomedical waveform signal being compressed for local storage following the capture and decompressed when later being extracted from the local storage. In some embodiments, the intermediate circuit/function 105 may be suitable both for storage and distribution. For example, a captured biomedical waveform signal may be stored on a memory card, such as an SD card, or USB device or any other removable storage medium, which can then be removed and provided to a different device where the data signal is extracted from the memory card and decompressed to provide a reconstructed biomedical waveform signal.

In order to reduce the storage and/or communication demands for the intermediate circuit/function 105, it is desired that the data size/rate of the data signal is as small as possible, yet that it still allows a sufficiently accurate reconstruction of the biomedical waveform signal. Accordingly, it is desired that the compression and decompression processes are as efficient and accurate as possible. As such it is desired that the generated data signal provides an efficient data representation that supports such efficient compression and decompression operations. Further, in order to facilitate the implementation and operation, it is desired that the data signal allows an efficient representation and a suitable selection of data and parameters. Accordingly, a suitable selection and structure of data for the data signal is desired to improve the overall performance.

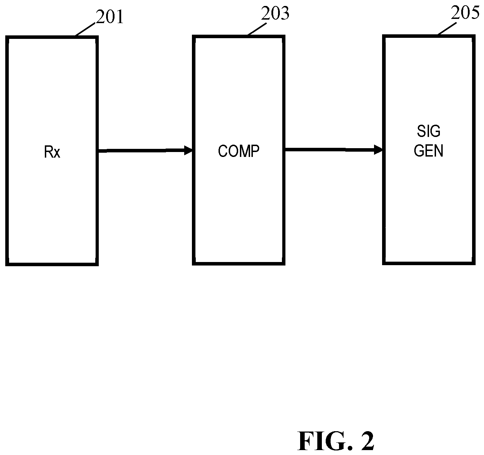

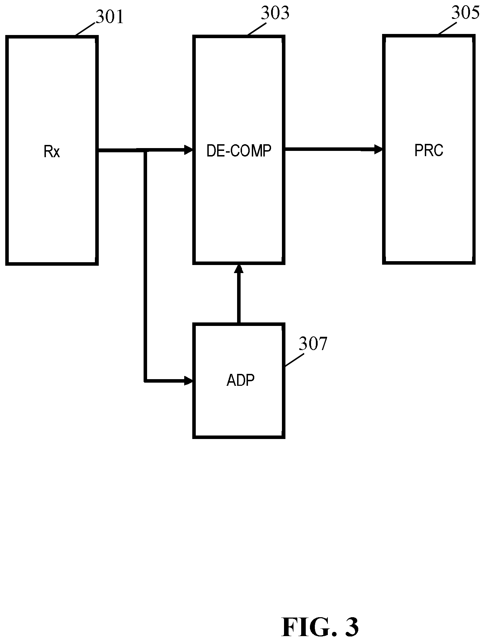

FIG. 2 illustrates an example of some elements of the compression device 101 of FIG. 1 and FIG. 3 illustrates an example of some elements of the decompressor device 107 of FIG. 1.

The compression device 101 comprises a receiver 201 which receives the biomedical waveform signal from the sensor 103. The receiver 201 is coupled to a compressor 203 which is arranged to perform a compression process to generate compressed data from the biomedical waveform signal. The compression may be an encoding/compression which results in a representation of the biomedical waveform signal which has a reduced data rate/size relative to the biomedical waveform signal received from the sensor 103. As will be described in more detail later, the compression may in some cases be a lossy compression and may in some cases be a lossless compression.

The compressor 203 is coupled to a data signal generator 205 which is arranged to receive the compressed data for the biomedical waveform signal and to generate a data signal that includes the compressed data. As will be described further later, the data signal may also include other data such as data describing other signals or other auxiliary data.

The data signal may then be stored/retrieved and/or transmitted/communicated in order to be provided to the decompressor device 107. The decompressor device 107 accordingly comprises a receiver 301 which receives the data signal. The receiver 301 is coupled to a decompressor 303 and is arranged to extract the compressed data from the received data signal and provide it to the decompressor 303.

The decompressor 303 is arranged to perform a decompression process on the compressed data to generate a reconstructed biomedical waveform signal. If the compression/decompression is ideal and lossless (and no data errors are introduced by the storage/communication), the reconstructed biomedical waveform signal will be identical to the biomedical waveform signal input to the compressor 203. However, in many cases, in order to provide a sufficient reduction in the data rate by the compression approach and/or to maintain low complexity/resource usage, the reconstructed biomedical waveform signal may often deviate from the original biomedical waveform signal but will typically be a sufficiently close approximation thereto, Such approximations will typically still allow the signal to be used for further applications, such as subjective clinical evaluation for detecting anomalies or feature extraction processing, e.g., deriving the heart-rate or breathing-rate from an PPG signal, etc.

The decompressor 303 is coupled to a processor 305 which is fed the reconstructed biomedical waveform signal, and which is arranged to perform a processing of the reconstructed biomedical waveform signal. Many different approaches and techniques based on a biomedical waveform signal are known and the use/processing of the biomedical waveform signal depends on the individual embodiments and indeed on the specific nature of the biomedical waveform signal, and in particular on the type of the biomedical waveform signal. For example, in many embodiments, the biomedical waveform signal may be processed such that the waveform can be displayed on a suitable display. In other scenarios, the reconstructed biomedical waveform signal may be analyzed to extract statistical properties, may be compared to other signals (specifically other biomedical waveform signals), etc.

In the approach, improved compression/decompression may typically be achieved, and specifically the described system employs a data signal that enables the compression device 101 to control and adapt the decompression performed by the decompressor device 107. In particular, the compression device 101 is arranged to generate the data signal to comprise an indication of a property of a compression process used to generate the compressed data from the biomedical waveform signal. The indication of the property of the compression process, henceforth for brevity referred to as the compression property indication, may for example indicate the compression algorithm that was used, or a specific parameter value or setting that was employed, for the compression of the biomedical waveform signal.

The decompressor device 107 comprises an adapter 307 which is coupled to the receiver 301 and the decompressor 303. The receiver 301 may extract the compression property indication and feed it to the adapter 307. The adapter 307 is arranged to adapt the compression process performed by the decompressor 303 dependent on the compression property indication. For example, the adapter 307 may, based on the compression property indication, select a corresponding decompression algorithm or set a corresponding parameter of the decompression process performed by the decompressor 303. Accordingly, the decompression may be adapted/improved/optimized in accordance with the instructions/directions provided by the compression device 101 by the compression property indication included in the data signal. Thus, not only does the data signal provide the compressed data itself, but it also allows far end control/adaptation of the decompression algorithm. This may allow substantially improved compression/decompression in many scenarios and embodiments. For example, it may provide increased flexibility and freedom in adapting, modifying, and optimizing the compression process thereby enabling an improved and more efficient compression resulting in a reduced data size/rate versus quality/accuracy of the compression/decompression.

Different compression/decompression algorithms may be used in different embodiments, and indeed in some embodiments multiple different algorithms may be used in different scenarios and/or for different biomedical waveform signals and/or for parts of. However, in many embodiments, the compression process may specifically employ a wavelet transform and the compression may be a wavelet compression algorithm/process. Correspondingly, the decompression may perform a wavelet decompression algorithm. Thus, in many embodiments, the compressed data includes wavelet transform coefficient data generated by wavelet compression, and the decompression may apply a wavelet decompression algorithm to the wavelet transform coefficient data. The Inventors have realized that a wavelet compression/decompression approach as described is particularly suitable and advantageous for compression and decompression of biomedical waveform signals, and in particularly for compression and decompression of an electrocardiography (ECG), electroencephalography (EEG), electromyography (EMG), or photoplethysmography (PPG) signal (and indeed for a number of other biomedical signals).

Indeed, wavelet transforms, and in particular the Discrete Wavelet Transform (DWT), are well suited for processing of signals of physical nature (time domain, discrete type) that can be found in real-life.

A DWT may split the time-domain signal into a low frequency band using a filter g0[n] and a high frequency band using a filter h0[n]. The low-band filter g0[n] and the high-band filter h0[n] are typically complementary filters such that these constitute a critically sampled filter-bank. As a result, the outputs of the analysis filters g0[n] and h0[n] may be critically sampled (decimated by a factor 2) such that the sampling rate at the input time-domain signal x[n] is the same as the (combined) sampling rate at the outputs of the analysis filters g0[n] and h0[n]. There are thus no additional samples (data) generated at the output of the analysis filter bank. Using a complementary synthesis structure with filters g1[n] and h1[n], perfect or near perfect reconstruction may be obtained. In the case of near-perfect reconstruction, the error after the reconstruction falls below a certain threshold, typically the selected resolution of the time-domain representation of the biomedical signals, such as 16 or 24 bits resolution, but also other thresholds may be used, such as frequency dependent thresholds to provide for a frequency dependent reconstruction error. The choice of the wavelet filters provides a degree of freedom in the design of the DWT. Critical sampling of filter banks as well as (near) perfect reconstruction is well known from the audio coding domain using critically sampled filter banks such as Quadrature Modulate Filter (QMF) or Modulated Discrete Cosine Transform (MDCT) filter banks. Typically, the filter banks used in the audio coding domain use much more than 2 filters, for example 32 or 64.

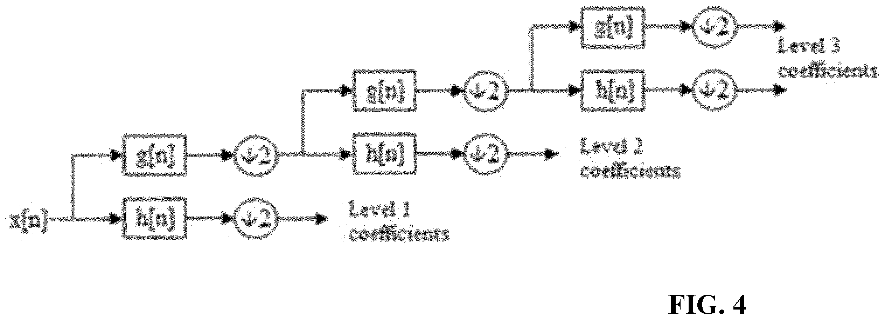

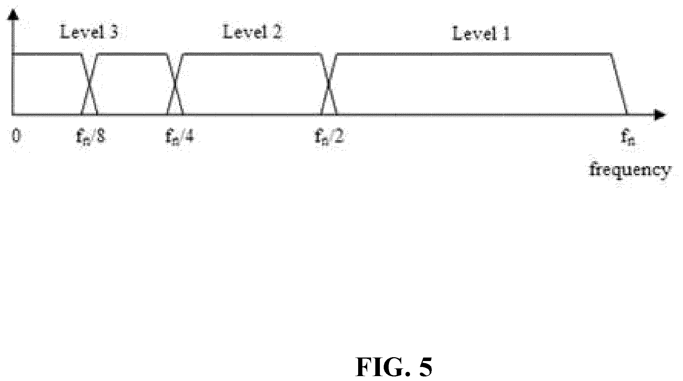

As illustrated in FIG. 4, in a DWT, for each subsequent level, only the low band is typically further split using the same analysis filter g0[n] (in the figure indicated simply by the reference g[n] and with h0[n] being indicated by h[n]), resulting in a further split of the low frequency band until eventually a filter tree up to a level J is obtained (see Fig. 5). In the context of wavelet filterbanks, the LF (low frequency) portions are denoted as 'Approximation coefficients'. The HF (high frequency) portions are denoted as 'Detail coefficients'. Detail coefficients can be seen as what is missing from the approximation at level J to get to the approximation at level J-1.

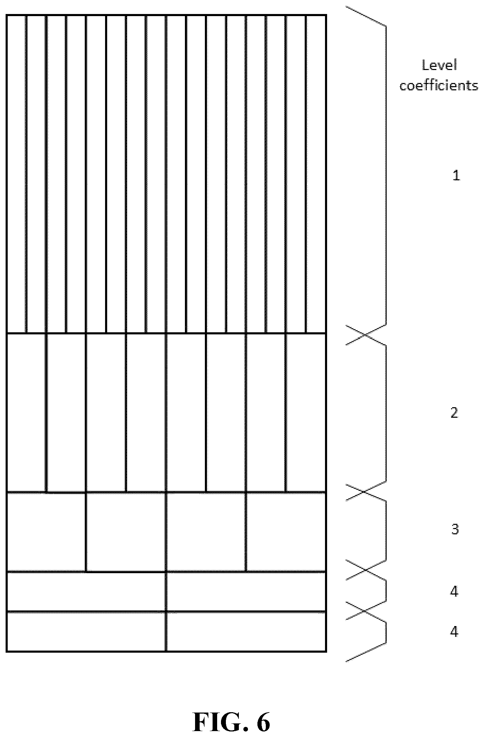

When applying a DWT onto a time domain signal, it produces a set of coefficients that represent specific time-frequency tiles as illustrated in FIG. 6, with time on the horizontal axis and frequency on the vertical axis, where each block represents a specific time-frequency interval, i.e., an area bounded by a specific time and frequency interval. In practice, the 'bounding' is not hard because of the non-perfect frequency resolution of practical filters, i.e., in practical implementations, filters are of finite length and therefore brick-wall filters are not feasible. Low frequency coefficients have a high frequency resolution but a low time resolution. On the other hand, high-frequency coefficients have a low frequency resolution but high time resolution (see Heisenberg boxes/rectangles in time-frequency representations). This combination of frequency and time resolution is compared to match well to physical processes. In comparison, general subband- or frequency-transform filter banks typically employ a uniform division of the time/frequency plane.

The DWT may be applied to subsequent segments of a signal so that a block-based processing is enabled. This is particularly useful for use cases that require random access into the compressed representation (bitstream) representing the biomedical signal.

Typically, the compressed data may be encoded and decoded as part of being included in the data signal and being extracted therefrom. Thus, the compressed data of the data signal may be encoded compressed data.

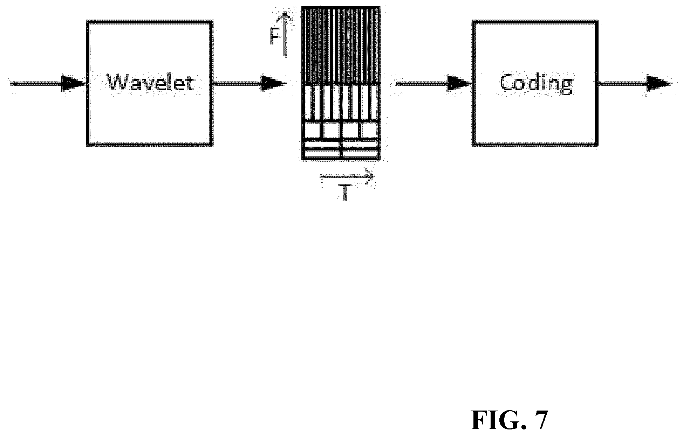

For example, as illustrated in FIG. 7, an approach based on wavelet transforms. In the example, the generated wavelet coefficients are further compressed by an encoding that includes a data compression. Typically, this encoding may comprise different techniques including:

- Removal of redundancy - lossless coding techniques such as prediction, entropy coding, ...
- Removal of irrelevancy - lossy coding techniques such as quantization, noise substitution, ...

Redundancy removal may preserve the perfect reconstruction of the input signal. After irrelevancy removal, perfect reconstruction is generally no longer feasible.

Exploiting redundancy may for example include encoding parts of the biomedical waveform signal/some wavelet coefficients relative to other parts of the biomedical waveform signal/wavelet coefficients. Principally, the removal of irrelevance is often obtained by e.g., simple thresholding (quantization) (i.e., putting to zero coefficients less than a given threshold) thanks to the relevant property of the wavelet transform of concentrating the most relevant information of the original signal resides in the biggest (by absolute magnitude) coefficients.

For entropy coding various techniques and methods are available, such as Huffman coding or Arithmetic coding. Of importance to the global efficiency of the entropy compression are also techniques that try to separate the signal into well-behaved (i.e., uniform) components like splitting the sign and mantissa, delta coding etc., of the wavelet coefficients.

The bitrate versus quality performance in lossy compression is largely determined by the error measure that is applied and used to adapt the trade-off. For example, when quantizing the coefficients representing the time-frequency tiles, the number of bits allocated to the respective quantizers not only determines the bitrate but also the resolution or quality with which the coefficients are represented. Ideally, the available bit-budget should be distributed over the time-frequency tiles such that the quality of the reconstructed signal is maximized for the configured bitrate.

In evaluating the performance of biomedical signal compression, two main error measures are often employed to represent an indication of the reconstructed signal quality. These error measures are Channel-normalized percentage root mean square Difference (or Distortion) (CPRD) and Peak Signal to Noise Ratio (PSNR).

$$CPRD = \frac{100\%}{N} \cdot \sum_{i=0}^{N-1} \sqrt{\frac{\sum_{j=0}^{M-1}\left(a_{i,j} - \tilde{a}_{i,j}\right)^2}{\sum_{j=0}^{M-1}\left(a_{i,j} - m_i\right)^2}}$$

$$PSNR_i = -10 * \log_{10}\left(\frac{MSE_i}{(2^B - 1)^2}\right)$$

CPRD takes into account variance-ranges in the different channels as well as the mean value for each channel and is represented as a percentage. PSNR represents the ratio between the power of the input signal and the power of the noise in decibels.

In many embodiments, the compression property indication may be indicative of a parameter of the compression algorithm, and specifically may be indicative of a compression parameter of the wavelet compression. The adapter 307 may proceed to adapt the wavelet decompression to use a corresponding parameter.

In many embodiments, the compression employs a wavelet transform and the compression property indication is advantageously an indication of a mother wavelet function for the wavelet transform. The adapter 307 may accordingly proceed to adapt the applied wavelet decompression algorithm and specifically may be arranged to adapt the decompression to use the mother wavelet as indicated by the compression property indication in the data signal.

In some embodiments, the compression property indication may alternatively or typically additionally to the indication of the mother wavelet, including an indication of a father wavelet for the wavelet transform. A father wavelet may also be referred to as a scaling function and may together with the mother wavelet describe/define the wavelet of the wavelet transform. As an example, in the previous description of a wavelet transform, the 'mother wavelet' may define the filter g[n], whereas the 'father wavelet' may define the filter h[n].

A mother wavelet may be a polyharmonic wavelet function that serves as a basis for generating other wavelets through scaling and translation operations. The term "mother wavelet" is in the field typically used to describe its role as the foundational function for generating a (typically complete) set of wavelets. The mother wavelet function may specifically be the mathematical function that represents a finite-length (compact) waveform which is translated and scaled for the different levels/coefficients.

In more detail, wavelets are typically a class of functions used to localize a given function in both space and scaling. A family of wavelets can be constructed from a function, referred to as the mother wavelet, which is confined in a finite interval. A wavelet family is a collection of functions obtained by shifting and dilating the graph of a wavelet. The wavelet family with mother wavelet $\psi$(t) may consist of functions $\psi_{a,b}$(t) of the form $\psi_{a,b}(t) = \frac{1}{\sqrt{a}} \psi\left(\frac{t-b}{a}\right)$, where b is the shift or center of $\psi_{a,b}$(t), and a is the scale.

The flexibility provided by the choice of the mother wavelet allows an additional degree of freedom in the DWT design. The approach may support selection of the mother wavelet depending on the signal type and characteristics, thereby optimizing the resolving properties of the DWT as a function of the signal type and characteristics on a channel/block basis. For additional flexibility in the approximation scheme the mother wavelet can be specified from an, optionally variable, list of appropriate functions.

Thus, in some embodiments, the compression property indication may be indicative of a mother wavelet from a set of mother wavelets, which specifically may be a predetermined set of mother wavelets. For example, the compression property indication may provide an index to a wavelet from the set of indexed wavelets.

The compression device 101 may be arranged to select the mother wavelet indicated by the compression property indication in dependence on a signal property/characteristic of the biomedical waveform signal. For example, in some embodiments, the compressor 203 may analyze the biomedical waveform signal and depending on the signal properties/characteristics, such as e.g., a periodicity or waveform, select a given mother wavelet. In other embodiments, the compressor 203 may for example select a given mother wavelet depending on the signal type of the biomedical waveform signal.

In some embodiments, the mother wavelet may specifically be defined by a user and thus the compression property indication may be of a mother wavelet determined in response to a user input.

In some embodiments, the compression property indication may indicate the mother wavelet by an encoding of the mother wavelet, such as an encoding of the actual waveform of the mother wavelet. In some embodiments, the encoding of the compression property indication may be a relative encoding, e.g., relative to a default or nominal wavelet function.

In some embodiments, the compression device 101 may be arranged to update the mother wavelet in time and specifically the data signal may be generated to include a compression property indication at repeating time intervals with each compression property indication indicating the mother wavelet to be used for a given time interval. Such an approach may provide a highly efficient and dynamic encoding/compression of a biomedical waveform signal.

In some embodiments, the compressor 203 may determine the mother wavelet as a mother wavelet from a set of predetermined mother wavelets. For example, the compressor 203 may perform a compression based on each of the mother wavelets of the set of predetermined mother wavelets and for each of the mother wavelets the resulting compressed data may then be used to reconstruct the biomedical waveform signal and this reconstructed biomedical waveform signal may be compared to the original signal and a quality measure may be determined based on how closely these match. The compressor 203 may then proceed to select the mother wavelet for which the desired trade-off between the size of the compressed data size and the quality measure is determined (or e.g., simply for which the best quality measure is detected). The compression property indication may then be generated to indicate the selected mother wavelet and the compressed data included in the data signal may be selected as the compressed data generated for the selected mother wavelet.

In some embodiments, all wavelets of the set of predetermined mother wavelets may be evaluated. In some embodiments, e.g., a subset of the mother wavelets may be evaluated with e.g. the subset being detected based on a property of the biomedical waveform signal.

In the approach, a DWT may thus be used to transform time domain digital biomedical waveform signals into a time-frequency domain such that redundancy and irrelevancy can be removed more efficiently in this transformed space than in the original captured digital signals. The DWT in addition has several parameters that can be tuned towards the characteristics of the signals to be compressed. One of the most important DWT parameters is the mother wavelet and in the described approach, this may be adapted by the compressor 203, with the selected mother wavelet being indicated in the data signal thereby allowing the decompressor 303 to perform the complementary operation (the decompression) using the selected mother wavelet.

This may provide improved performance and in particular may support dynamic changes by the data signal comprising multiple compression property indications which indicate changes of the mother wavelet during the compression of the data signal. For example, dependent on a change in the characteristics of the biomedical waveform signal, a better tailored time-frequency analysis may be performed by the mother wavelet for the wavelet transforms being dynamically adapted.

As another example, in some embodiments and scenarios, the compression requirements may change, e.g., upon system or user request, and a similar change in the mother wavelet may be implemented.

The data signal may include a compression property indication that indicates the choice of mother wavelet. The indication may be included in the data signal in accordance with a suitable representation which may specifically follow a given syntax. For example, a syntax may be used which allows the mother wavelet to be selected from a predefined set and/or to be custom defined and embedded in the data stream using a suitable syntax.

In some embodiments, the mother wavelet may by default correspond to one out of the predefined set of mother wavelets dependent on e.g. the type of the biomedical waveform signal (e.g. whether it is an ECG, EEG, EMG, or PPG signal).

The selected mother wavelet may e.g. be represented in explicit form or may e.g. be represented as a modification or derivation from a previously used custom mother wavelet, or a mother wavelet from the set of predefined mother wavelets.

A suitable syntax for data of a compression property indication to represent/indicate a suitable syntax may for example be the following:

| Syntax | No. of bits | Mnemonic |
|---|---|---|
| motherWavelet() | | |
| { | | |
| **isPredefined** | **1** | **bslbf** |
| if (isPredefined) { | | |
| **predefinedMotherWavelet** | **8** | **uimsbf** |
| } else { | | |
| **motherWaveletType** | **4** | **uimsbf** |
| motherWaveletCoefs() | | |
| } | | |
| } | | |

The decompressor device 107 may correspondingly extract the compression property indication and determine which mother wavelet is indicated by the compression property indication. It may then proceed to perform the wavelet decompression using the selected mother wavelet.

Alternatively or additionally, in some embodiments, the compression property indication may comprise an indication of a property of a decomposition tree for the wavelet transform. The compression property indication may specifically be indicative of a depth or level of the wavelet transform. The compression property indication may also be indicative of the splitting structure of the wavelet tree, e.g. representing alternative structures different from exclusively further splitting the low frequency portion.

The configuration of the discrete wavelet transform as represented by the depth or level of the tree has a significant impact on the compression performance including having an impact on parameters such as the compression efficiency, accuracy, and the computational complexity and resource usage. The compression device 101 may accordingly proceed to determine a suitable depth for the wavelet transform and then adapt the wavelet transform of the compression to be configured with such a depth. The compression device 101 may then include a compression property indication in the data signal to indicate the depth used.

The decompressor device 107 may perform the complementary/corresponding operation and may specifically proceed to extract the compression property indication and from this determine the indicated configuration/depth of the wavelet transform. It may then adapt the decompression process to perform the inverse wavelet transform operation by configuring the decompression process to use a corresponding depth/configuration.

The encoder may for example use a similar approach to determine the desired configuration/depth, e.g., it may proceed to perform and evaluate the compression for a number of potential depths and select the one resulting in the best performance in accordance with a suitable requirement/quality measure. As another example, the compression device 101 may proceed to select a depth based on a property of the biomedical waveform signal. For example, a mapping between a given property of the biomedical waveform signal and a preferred depth may be stored and used to select the depth.

In other embodiments, the compression property indication may be arranged to comprise other variable parameters of the wavelet transform, such as one or more of an adaptive frame/ block size/resolution. Such parameters may be adapted using a similar approach to that described above for selecting the mother wavelet and/or the depth level.

In some embodiments, the compression property indication may be arranged to comprise an indication of a time-frequency resolution of the (discrete) wavelet transform (such as e.g., indicated by the depth/level of the discrete wavelet transform). Such a compression property indication may for example be included in the data signal in accordance with the following syntax:

| Syntax | No. of bits | Mnemonic |
| --- | --- | --- |
| DWT(level) | | |
| { | | |
| motherWavelet() | | |
| If (level > 1) { | | |
| **If (approx_tree)** { | **1** | **bslbf** |
| DWT_approx = DWT(level-1) | | |
| } | | |
| If **(detail_tree)** { | **1** | **bslbf** |
| DWT_detail = DWT(level-1) | | |
| } | | |
| } | | |
| } | | |

In some embodiments, the compression property indication may be indicative of the compression algorithm that is used to generate the compressed data. The decompressor device 107 may then extract the compression property indication and select the decompression algorithm that is complementary to the indicated compression algorithm. It will be appreciated that in some embodiments, the compression algorithm used may be indicated by the compression property indication indicating which (complementary) decompression algorithm to use.

In many embodiments, the compression/decompression algorithms may be selected from a predetermined set of possible algorithms. For example, the encoder may be arranged to use a compression algorithm selected from a set of algorithms including for example a wavelet transform algorithm, a transform encoding algorithm, an entropy coding algorithm, etc.

Similarly to previously described, the compression device 101 may proceed to perform decompression using the different options and select the one resulting in the best approach in accordance with a suitable quality measure.

In some embodiments, the compression device 101 may be arranged to select the compression algorithm(/decompression) in dependence on the biomedical waveform signal. For example, the compression algorithm may be selected depending on the biomedical waveform signal type (e.g., whether it is an ECG, EEG, EMG, or PPG signal). As another example, it may be selected based on e.g., a variation measure or periodicity property of the biomedical waveform signal. In some embodiments, the compression algorithm may for example be selected based on the number of biomedical waveform signals that are included in the data signal (each biomedical waveform signal may be considered a channel, and the compression algorithm may be selected based on the number of channels to be encoded in the data signal). In some embodiments, the selection may for example be made dependent on a requirement for the data signal, such as e.g., dependent on the required data size/rate of the biomedical waveform signal.

In some embodiments, only one compression property indication may be provided for the biomedical waveform signal. In some embodiments, the compression property indication may be a static compression property indication that applies to the entire biomedical waveform signal in the data signal. For example, the compression property indication may indicate which compression algorithm has been used for compressing the entire biomedical waveform signal.

In some embodiments, the data signal may comprise a plurality of compression property indications with the (or at least some) compression property indications being linked with different time intervals. The compression device 101 may for example at regular time intervals determine a suitable compression parameter value or e.g., compression algorithm resulting in the compression parameter or algorithm potentially changing during the biomedical waveform signal time evolution. For example, the periodicity of an ECG feature (e.g., QRS complex etc.) may change during the capture of the ECG biomedical waveform signal and the wavelet properties (such as e.g., size/duration/frame size/block size/ depth level) may be dynamically changed to reflect this new periodicity. Accordingly, whenever the wavelet properties (e.g., size/duration/frame size/block size/ depth level) are changed, a compression property indication may e.g., be included in the data signal to indicate the new wavelet properties (e.g., size/duration/frame size/block size/ depth level).

The adapter 307 of the decompressor device 107 may extract the plurality of compression property indications and dynamically adapt the decompression accordingly. The adapter 307 may for each time interval adapt the decompression process to employ the approach/parameter indicated for the compression property indication linked with that time interval.

The linking between time intervals and compression property indications may be explicit such as by each compression property indication specifically indicating a time interval to which it applies. In other embodiments, the indication may alternatively or additionally be implicit. For example, the compression property indications may be inserted into the compressed data bitstream for the biomedical waveform signal such whenever a change occurs, a compression property indication is inserted into the compressed data bitstream to indicate the decompression parameter values that are to be applied from then on and until the next compression property indication is received.

The compression property indications may in some embodiments relate to different parameters and the time intervals may be overlapping time intervals. However, in many embodiments, some or more of the compression property indications may be for the same compression/decompression parameter and a given compression property indication may indicate that (and how) a given parameter should change relative to the value provided by another compression property indication. In many embodiments, the time intervals linked with different compression property indications may accordingly be disjoint time intervals and/or different compression property indications may indicate different values for the same compression/decompression parameter.

The approach may in many scenarios provide a dynamic and flexible adaptation of the compression and decompression operation with a close interworking between the source (compression) end and the destination (decompression) end.

In some embodiments, the data signal may include data for at least one further signal.

The data representing the further signal may typically be compressed data, and in particular the data for the further signal may typically be compressed/encoded data representing the signal.

In some embodiments, the further signal may be another biomedical waveform signal and indeed may be another signal measured as part of the same measurement. For example, in many embodiments, the data signal may include a plurality of biomedical waveform signals that are all related to the same underlying biomedical measurement. For example, the data signal may include a biomedical waveform signal for each electrode/sensor of a measurement that includes a plurality of such electrodes/sensors. For example, for an ECG or EEG measurement, a plurality of electrodes is in contact with the test subject/patient and a plurality of signals is measured together. In many embodiments, the data signal may include all such signals from a measurement with each of these biomedical waveform signals being compressed and represented by compressed data in the data signal. Such different biomedical waveform signals may also often be referred to as different channels, and specifically as different channels of the biomedical measurement.

As an example, each captured biomedical waveform signal of a measurement may individually be compressed as previously described for a single biomedical waveform signal. The resulting compressed data for all biomedical waveform signals may then be included in the data signal. However, in some embodiments, some cross signal/cross channel compression may be applied to provide a more efficient encoding of the biomedical waveform signals. For example, one biomedical waveform signal may be encoded relative to another biomedical waveform signal such as e.g., by compressing it as a difference signal with respect to the other biomedical waveform signal. Another technique is to use a prediction filter to predict one biomedical waveform signal relative to one or more other biomedical waveform signals and compressing the prediction error signal with respect to the other biomedical waveform signals. Next to this prediction error signal, the prediction coefficients may be fixed or transmitted as a selection from a set of prediction coefficients or transmitted by their coefficient values.

In some embodiments where the data signal comprises a plurality of biomedical waveform signals, the data signal may include compression property indications that are individual to each of the biomedical waveform signals. Alternatively or additionally, the data signal may include one or more compression property indications that are common to at least two of the biomedical waveform signals. For example, for different channels of a corresponding ECG or EEG measurement, the biomedical waveform signals tend to be similar and the same compression algorithm/parameter settings may typically be suitable for all of the biomedical waveform signals. For example, a compression property indication may indicate a mother wavelet that may be used for the (typically discrete) wavelet transform for all the biomedical waveform signals.

The decompressor device 107 may proceed to reconstruct all the biomedical waveform signals using the compression property indications of the data signal as previously described. In embodiments, where a compression property indication is included that is common for a plurality of biomedical waveform signals, the adapter 307 may determine the corresponding parameter value and then apply it to the decompression of all the biomedical waveform signals to which it applies.

In some embodiments, the further signal may be a non-biomedical waveform signal. For example, the data signal may further include one or more accelerometer signals, signals relating to the environment, audio signals as well as e.g., diagnostically relevant sensors data/signals, such as e.g., signals reflecting sweating, skin conductivity etc. The data signal may in some cases include other signals that are convenient or practical to include in the data signal e.g., simply due to it providing potentially relevant information that e.g., may be useful for diagnostic purposes. Alternatively or additionally, the data signal may include some signals that are correlated with one or more biomedical waveform signals although not directly part of the same measurement. For example, an accelerometer positioned appropriately on a patient may generate a waveform that has common properties with the biomedical waveform signals. For example, an accelerometer positioned near the heart of a person may generate a signal that has a related periodicity to the biomedical waveform signals measured as part of an ECG measurement.

In many embodiments, the further signal may accordingly be a measurement of a different modality than the biomedical waveform signal and may specifically be a different type of signal.

In some cases, the compression device 101 may be arranged to utilize such signals in the compression of the biomedical waveform signals and the decompressor device 107 may similarly be arranged to utilize the signals in the decompression to reconstruct the biomedical waveform signals.

In some embodiments, the data signal may include data that is indicative of a relationship between the different signals, and specifically the data signal may include a compression property indication which is indicative of a grouping of the signals of the data signal.

For example, a grouping indication may be included which indicates that a set of the signals of the data signal relates to the same measurement, and/or e.g., that they reflect simultaneous measurements.

In some embodiments, the grouping indication may be included to indicate that a given biomedical waveform signal and another signal belong to the same group. The grouping indicator may indicate a group of signals including one or more biomedical waveform signals. In some embodiments, all the signals of the group may be biomedical waveform signals. For example, a grouping indication may indicate all the signals/channels of a given multi signal/channel measurement, such as an ECG or EGG. In some embodiments, at least one of the signals of the group may be a non-biomedical waveform signal. For example, it may be a measurement of a signal performed at the same time as the measurement of the biomedical waveform signal(s).

The decompressor device 107 may extract the grouping indication and proceed to adapt the decompression dependent on the grouping indication. For example, the decompressor 303 may proceed to perform a joint decompression of the signals indicated by the grouping indication to belong to the same common group.

The changes of parameter values are not limited to e.g., changes in signals characteristics over time, but may also be applied to changes in grouping, e.g., signals may be added or removed from a given group. The latter may happen when signal sensors have been activated or deactivated temporarily. When grouping signals, they may either be interlaced/ interleaved, or simply appended to each other depending on the method of compression. In some embodiments, a plurality of grouping indications may be provided with these being specific to a given time interval. For example, an approach corresponding to that previously described for multiple compression property indications may be used.

The approach may thus employ a compression approach that supports different biomedical signal modalities (ECG, EMG, EEG, PPG, etc.) and which provides flexible and efficient syntax signaling of different modalities. This includes the support for so called 'sub-signals'. For example, PPG signals may be represented as biomedical waveform signal + accelerometer data + ambient signal. In addition, diagnostically relevant sensors' signals, e.g., sweating, skin conductivity etc. may be included. The approach may also support custom signals (beyond ECG, EMG, EEG, PPG) to make the compression scheme future proof.

A signal index and signal type may be provided for each signal, and this can be used to create groups within one modality and/ or over modalities. An example of grouping within one modality is to combine sensor signals corresponding to a specific region (e.g., according to the standard International 10/20 system) of an EEG recording. Two examples of grouping over modalities are a subset of ECG leads in combination with a PPG signal and a region of EEG signals in combination with ECG, heart rate, accelerometer and oxygen saturation signals.

An example of a syntax supporting grouping over modalities may be the following:

| Syntax | No. of bits | Mnemonic |
|---|---|---|
| Grouping() | | |
| { | | |
| **numGroups** | **8** | **uimsbf** |
| for (int g = 0; g < numGroups; g++) { | | |
| group Type | **8** | **uimsbf** |
| numGroupChannels[g] | **8** | **uimsbf** |
| for (int n = 0; n < numGroupChannels[g]; n++) { | | |
| groupChannel[g] [n] | **8** | **uimsbf** |
| } | | |
| } | | |
| } | | |

In this example, each signal index may be included in at most one group. Signal indices which are not included in the grouping correspond to signal channels which are considered as a separate group, similar to groups which contain only a single member.

In a hybrid mode, grouping can also be used to switch between coding modes. Some parts of the signals may require a higher resolution than other parts of the signals and lossless coding of those parts may be needed to fulfil such a requirement. For example, the QRS complex in an ECG signal could be compressed/coded lossless, whereas the rest is compressed/coded lossy (if there is no event detected). In such a hybrid mode, lossless and different types of lossy coding can be used interchangeably. Next to the use of the DWT, other transformations, e.g. DFT, DCT or MDCT can be used.

A possible syntax for such a hybrid approach may be:

| Syntax | No. of bits | Mnemonic |
|---|---|---|
| hybridCoding() | | |
| { | | |
| **hasHybridCoding** | **1** | **bslbf** |
| if (hasHybridCoding) { | | |
| **numTimeSegments** | **5** | **uimsbf** |
| for (i=0; i<numTimeSegments; i++){ | | |
| **signalCodingTimeBoundary[i]** | **5** | **uimsbf** |
| **signalCodingType[i]** | **4** | **uimsbf** |
| } | | |
| } | | |
| } | | |

In some embodiments, the data signal comprises compressed data for a plurality of biomedical waveform signals and a type indicator indicating the biomedical waveform signal type for at least some of the biomedical waveform signals. The type indicator may for example be arranged to indicate a type of the biomedical waveform signal where the possible types that can be indicated by the type indicator includes at least one of an ECG, EEG, EMG, or PPG.

The different types of the biomedical waveform signal may specifically be different modalities and thus the type indicator may specifically indicate a modality of the biomedical waveform signal for at least some of the biomedical waveform signals.

The type indicator may typically be included in a header portion of the data signal. A specific example of a header is indicated below. In the example, for each signal (channel) an index (i) and signal type (signalType) are associated, and signal type specific information is included, e.g., scaling and sampling rate. The approach may provide a flexible and efficient syntax signaling of the different modalities.

| Syntax | No. of bits | Mnemonic |
|---|---|---|
| compressedMedicalSignal() | | |
| { | | |
| **versionNumber;** | **16** | **uimsbf** |
| **hasPatientData;** | **1** | **bslbf** |
| If (hasPatientData) { | | |
| readPatientData() | | |
| } | | |
| maxNumSamplesPerFrame | **20** | **uimsbf** |
| startTime | **32** | **uimsbf** |
| **numChannels** | **8** | **uimsbf** |
| for (int i = 0; i < numChannels; i++) { | | |
| signalType | **4** | **uimsbf** |
| if (signalType == 'ECG') { | | |
| digitalMin | **4** | **uimsbf** |
| digitalMax | **4** | **uimsbf** |
| analogueMin | **4** | **uimsbf** |
| analogueMax | **4** | **uimsbf** |
| quantization[i] = LUT(signalQuantization) | **var** | **vlclbf** |
| **sampleRate** = getFs() | **4** | **uimsbf** |
| startingDCOffset | **20** | **simsbf** |
| } | | |
| } | | |
| | | |
| while (bitsAvailable() != 0){ | | |
| readFrame() | | |
| } | | |
| } | | |

In some embodiments, the possible compression/decompression algorithms that may be indicated by the compression property indication may include at least one lossy algorithm and at least one lossless algorithm.

Some use cases may not allow any degradation of the biomedical waveform signal in order to sufficiently accurately retain the specific clinical features (which are possibly not even known at the data of capturing) and ensure that they are not affected by the effects of the compression/ quantization. Therefore, the compression scheme needs to support a lossless mode. However, in other cases, it may be desirable to perform lossy compression/decompression in order to reduce the data rate further.

The approach may provide a hybrid mode that allows for flexible combination of lossy and lossless coding. Specifically, lossless coding may be applied to (manually or automatically) detected/ annotated segments comprising clinical features such as e.g., arrhythmia or seizures. The hybrid selection may be supported both in time as well as over channels that may contain signals of the same or different modality. The combination of the abovementioned compression modalities (lossless, lossy) enables the possibility for the approach to operate in the so-called near-lossless modality in which the allowed degradation of the original signal is much lower than what is achievable from the best lossy compression on the same data while obtaining, at the same time, a better compression ratio than the lossless compression.

In some embodiments, the data signal further comprises a timing synchronization indicator which is indicative of a timing difference/offset between the biomedical waveform signal and one or more of the other signal(s).

Such a timing indication may for example be provided once to indicate an overall timing offset and to allow an overall timing synchronization between the signals. In some embodiments, a plurality of timing synchronization indicators may be included. For example, timing synchronization indicators may be provided e.g., periodically or intermittently in the data signal. This may allow the decompressor device 107 to resynchronize the signals at different times.

The approach may provide for synchronization measures, and specifically between biomedical waveform signals, or between a biomedical waveform signal and other types of signals. Specifically, the approach may support synchronization between signals of different modalities. This not only enhances the correlation between the signals for exploiting redundancy (e.g., when applying difference coding or prediction coding between channels), but the synchronization information may also provide a clinician with meta-information on the alignment between the signals. The approach could also support alignment to other clinical data streams such as video.

With respect to synchronization of the different signals, the data signal may for example include data according to a syntax that may both define the relative, potentially time-varying, offset in clocks of the differently captured signals, and/or the relative delay of clinical events in the signals of the same or different modality. Distinct and different capture devices may use clocks that may drift over time. Signals captured at different locations on a patient body may have time and phase differences due to signal propagation through the patient's body. Signals of different modalities may have time and phase differences as propagation of e.g., blood and electrical signals is quite different through a patient's body. Such timing variations/differences may be represented by the timing synchronization indicator.

The adapter 307 of the decompressor device 107 may be arranged to extract the timing synchronization indicator and may proceed to align the signals in time by applying a time compensation to at least one of the signals based on the timing offset indicated by the timing synchronization indicator. Typically, a suitable delay may be applied to one of the signals with the delay being dependent on the timing synchronization indicator.

A suitable syntax for the timing synchronization indicator of the data signal may be:

| Syntax | No. of bits | Mnemonic |
|---|---|---|
| timeSync() | | |
| { | | |
| **hasTimeSyncData** | **1** | **bslbf** |
| if (hasTimeSyncData){ | | |
| for (i=1; i<numSignals;i++){ | | |
| if (**signalHasTimeSync**){ | **1** | **bslbf** |
| **delay[i]** | **5** | **uimsbf** |
| **compressionFactor[i]** | **5** | **uimsbf** |
| } | | |
| } | | |
| } | | |
| } | | |

In some embodiments, the data signal may further comprise an indication of an average level of at least part of the biomedical waveform signal. The decompression may then be arranged to generate the reconstructed biomedical waveform signal by taking this average level into account. For example, in some embodiments, the reconstruction of the biomedical waveform signal may include subtracting the indicated average level or e.g., it may include an offsetting or scaling to generate a reconstructed biomedical waveform signal that has an average value, which matches that indicated by the average level specified in the data signal.

The average level may be an average level for the entire biomedical waveform signal or may e.g., for only part, e.g., a time interval, of the biomedical waveform signal. The average level may e.g., be determined by applying a suitable low pass filtering to the biomedical waveform signal.

In some embodiments, the decompression algorithm may include generating the reconstructed biomedical waveform signal to have an average level matching the average level indicated by the indication of an average level.

In some embodiments, the decompression algorithm may compensate the reconstructed biomedical waveform signal for an average level matching the average level indicated by the indication of an average level. (specifically subtract the average level).

Some biomedical signals comprise a DC component which may fluctuate over time, and which may provide valuable clinical information (for example a breathing rate may be extracted from the DC component in an ECG signal). It is therefore advantageous that the DC component (including variations over time) is sufficiently retained. The described approach may support this.

The DC component of some biomedical signals can be quite substantial in (absolute) value and can lead to significant variations in the performance of the compression method. The approach may include explicitly representing the DC component in the data signal, and the decompressor device 107 may be arranged to apply a preprocessing to remove the DC component for such signals. The separate representation of the DC component not only benefits the compression efficiency (as it reduces the absolute signal values), but can further be beneficial for a clinician or intelligent algorithm to extract valuable clinical information, e.g., the breathing rate may be extracted from the DC component in an ECG or PPG signal.

A suitable syntax for such an indication of an average level may be:

| Syntax | No. of bits | Mnemonic |
|---|---|---|
| dcComponent() | | |
| { | | |
| for (int i=0; i < num Channels; i++) { | | |
| **numTimePoints** | **8** | **uimsbf** |
| **extrapolationMode** | **3** | **uimsbf** |
| for (int j=0; j < numTimePoints; j++) { | | |
| **dcComponentTimePoints[i] [j]** | **8** | **uimsbf** |
| **deComponent [i] [j]** | **8** | **uimsbf** |
| } | | |
| } | | |
| } | | |

In some embodiments, the compression property indication may indicate segments/time intervals of the biomedical waveform signal for which no data is included in the compressed data. Thus, for some segments/time intervals, the data signal does not include any compressed data representing the biomedical waveform signal.

The compression device 101 may specifically exclude compressed data for some segments/time intervals. For example, the compression device 101 may determine such segments/time intervals as segments/time intervals for which the amplitude of the biomedical waveform signal is below a given threshold, for which a signal to noise ratio is below a given threshold, for which there is no valid measurement, and/or which have been classified as having no significant clinical relevance, etc.

The compression property indication may indicate such segments/time intervals and the decompressor device 107 may accordingly adapt the decompression, e.g., to not generate any biomedical waveform signal for these segments/time intervals or by e.g., inserting a nominal or default e.g. predetermined signal.

The approach may support both time-continuous as well as time-discontinuous data such that only certain segments (episodes) or subsets of data-channels are retained in the compressed data format allowing, among others, for a very efficient storage as irrelevant portions of the signal can be discarded. These segments may be determined manually or even automatically, for example by setting an extremely pessimistic threshold such that the number of missed episodes is minimized.

In some embodiments, the data signal may comprise at least one event indication for the biomedical waveform signal where the event indication is indicative of a timing of an event. The event may for example be a clinical event or may e.g., be a signal event such as a time of a particular signal property (e.g., a peak value).

The data signal may e.g., support random access by adding a compression property indication in the form of meta data of different timescales which allows for searching backward/ forward from coarse to fine grid and searching based on (e.g., clinical) event annotations in the bitstream. It is also possible that the random-access points are limited to annotated locations, thereby minimizing the overhead for random access to the clinically relevant locations, but offering the clinician to quickly navigate back and forth between the annotated locations.

To support random access, a syntax may be used which identifies entry points with associated time stamps in the data streams, from which points instantaneous decoding is possible. The entry points may be associated with metadata to support improved clinical evaluation. The entry points can be used to zoom in on clinically relevant (time) locations and/or relevant (sub)groups of signals.

In some implementations it may be required to decode a number of time frames before reconstruction can begin. This may be due to inter-frame dependencies or by use of periodic independent frames (I-frames in video compression terminology), with P-frames (predicted frames) reliant on previous data in between the I-frames.

In some embodiments, the data signal further comprises at least one indication of a time interval for which the decompressor is arranged to generate at least a component of the biomedical waveform signal for the first time interval independently of the compressed data. The indication may specifically be of a time interval for which no data is included in the compressed data or for which the level of the biomedical waveform signal is below a given level etc.

The decompressor 303 may specifically be arranged to generate the biomedical waveform signal for the time interval using a predetermined waveform/signal component or e.g., may locally generate a waveform signal. For example, a random (noise) signal may be generated and used as the biomedical waveform signal for the given time interval.

Specifically for higher compression ratios, certain time/ frequency tiles may be reduced to a zero signal. Although this may result in a cleaner signal representation (small signal contributions that show as noise regions are removed), clinicians may not be used to diagnose such compressed/ decompressed signal representations. For that purpose, the approach may for example optionally support Comfort-Noise (CN) addition. Specifically, prior to compression, the spectral shape of the noise floor may be analyzed, parameterized and conveyed to the decoder. The decoder may then choose to synthetically reproduce a noise floor with the appropriate spectral shape at those regions that normally would be reduced to (close to) zero signal contributions.

To support higher compression ratios, noise substitution may be used. E.g., for signal parts that are identified as mainly noise, the spectral shape and energy level may be analyzed, parameterized and represented by a suitable syntax. Such noise components may be subtracted from the signal to reduce the compressed data size. In the decoder the noise components may be extracted from the received data stream and synthesized to be included in the reconstructing of the signals.

A suitable syntax may be:

| Syntax | No. of bits | Mnemonic |
|---|---|---|
| noiseSynthesis() | | |
| { | | |
| **noiseType** | **4** | **uimsbf** |
| **noiseGain** | **4** | **uimsbf** |
| **defaultFilter** | **4** | **uimsbf** |
| if (defaultFilter == 15) { // indicates custom filter | | |
| **highPassCutoff** | **5** | **uimsbf** |
| **highPassGain** | **5** | **uimsbf** |
| **bandPassCutoff** | **5** | **uimsbf** |
| **bandPassGain** | **5** | **uimsbf** |
| **lowPassCutoff** | **5** | **uimsbf** |
| **lowPassGain** | **5** | **uimsbf** |
| } | | |
| } | | |

The described approach may provide for a flexible bit-rate control processing that supports block-based operation. Specifically, bit-rate control may be flexible to adapt the compression depending on detected clinical events. Also, a clinical event detected in a specific channel may trigger the bit-rate control to not only adapt the rate for that channel, but also of the other channels, which may be the case for both channels of the same and of a different modality. Annotations of such events, including meta-data on the type of annotation (e.g., user/ automatically/ algorithmically triggered) may be included in the bitstream syntax.

Block-based processing may be used to enable flexible bit-rate control processing. Block sizes can be specified globally (for all signals) or for each (sub)group separately. The block size can be adapted depending on signal characteristics, compression requirements and/or clinical events present in the associated signal portion. Further, a table with compression rate per modality per desired quality level is proposed. Such a table may include compression rates depending on whether there is an annotation (e.g., clinical event, the addition or removal of a sensor, or the detection of a silent signal) detected and/or for how long that compression rate should remain.

The apparatus(es) may specifically be implemented in one or more suitably programmed processors. The different functional blocks may be implemented in separate processors and/or may e.g., be implemented in the same processor. An example of a suitable processor is provided in the following.

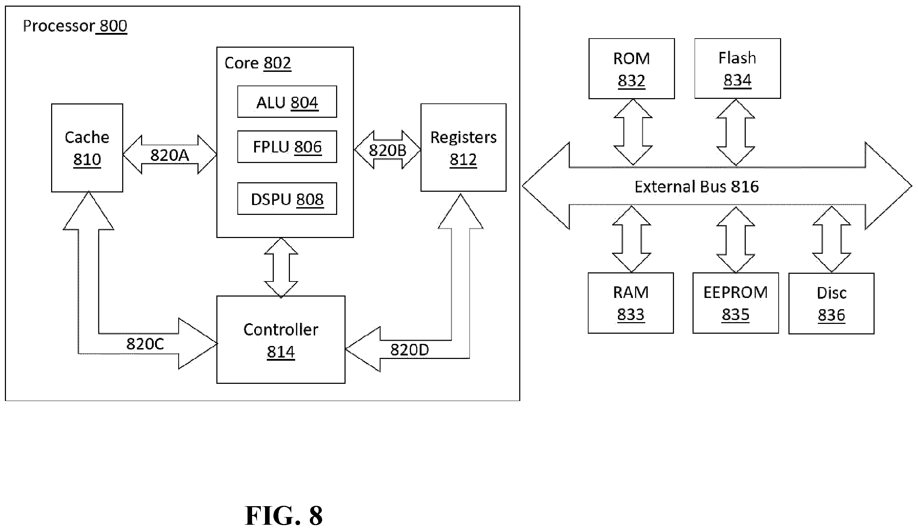

FIG. 8 is a block diagram illustrating an example processor 800 according to embodiments of the disclosure. Processor 800 may be used to implement one or more processors implementing an apparatus as previously described or elements thereof (including in particular one more artificial neural network). Processor 800 may be any suitable processor type including, but not limited to, a microprocessor, a microcontroller, a Digital Signal Processor (DSP), a Field ProGrammable Array (FPGA) where the FPGA has been programmed to form a processor, a Graphical Processing Unit (GPU), an Application Specific Integrated Circuit (ASIC) where the ASIC has been designed to form a processor, or a combination thereof.

The processor 800 may include one or more cores 802. The core 802 may include one or more Arithmetic Logic Units (ALU) 804. In some embodiments, the core 802 may include a Floating-Point Logic Unit (FPLU or FPU) 806 and/or a Digital Signal Processing Unit (DSPU) 808 in addition to or instead of the ALU 804 .

The processor 800 may include one or more registers 812 communicatively coupled to the core 802. The registers 812 may be implemented using dedicated logic gate circuits (e.g., flip-flops) and/or any memory technology. In some embodiments the registers 812 may be implemented using static memory. The registers may provide data, instructions and addresses to the core 802.

In some embodiments, processor 800 may include one or more levels of cache memory 810 communicatively coupled to the core 802. The cache memory 810 may provide computer-readable instructions to the core 802 for execution. The cache memory 810 may provide data for processing by the core 802. In some embodiments, the computer-readable instructions may have been provided to the cache memory 810 by a local memory, for example, local memory attached to the external bus 816. The cache memory 810 may be implemented with any suitable cache memory type, for example, Metal-Oxide Semiconductor (MOS) memory such as Static Random Access Memory (SRAM), Dynamic Random Access Memory (DRAM), and/or any other suitable memory technology.

The processor 800 may include a controller 814, which may control input to the processor 800 from other processors and/or components included in a system and/or outputs from the processor 800 to other processors and/or components included in the system. Controller 814 may control the data paths in the ALU 804, FPLU 806 and/or DSPU 808. Controller 814 may be implemented as one or more state machines, data paths and/or dedicated control logic. The gates of controller 814 may be implemented as standalone gates, FPGA, ASIC or any other suitable technology.

The registers 812 and the cache 810 may communicate with controller 814 and core 802 via internal connections 820A, 820B, 820C and 820D. Internal connections may be implemented as a bus, multiplexer, crossbar switch, and/or any other suitable connection technology.

Inputs and outputs for the processor 800 may be provided via a bus 816, which may include one or more conductive lines. The bus 816 may be communicatively coupled to one or more components of processor 800, for example the controller 814, cache 810, and/or register 812. The bus 816 may be coupled to one or more components of the system.

The bus 816 may be coupled to one or more external memories. The external memories may include Read Only Memory (ROM) 832. ROM 832 may be a masked ROM, Electronically Programmable Read Only Memory (EPROM) or any other suitable technology. The external memory may include Random Access Memory (RAM) 833. RAM 833 may be a static RAM, battery backed up static RAM, Dynamic RAM (DRAM) or any other suitable technology. The external memory may include Electrically Erasable Programmable Read Only Memory (EEPROM) 835. The external memory may include Flash memory 834. The External memory may include a magnetic storage device such as disc 836. In some embodiments, the external memories may be included in a system.

The invention can be implemented in any suitable form including hardware, software, firmware or any combination of these. The invention may optionally be implemented at least partly as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit or may be physically and functionally distributed between different units, circuits and processors.

Although the present invention has been described in connection with some embodiments, it is not intended to be limited to the specific form set forth herein. Rather, the scope of the present invention is limited only by the accompanying claims. Additionally, although a feature may appear to be described in connection with particular embodiments, one skilled in the art would recognize that various features of the described embodiments may be combined in accordance with the invention. In the claims, the term comprising does not exclude the presence of other elements or steps.

Furthermore, although individually listed, a plurality of means, elements, circuits or method steps may be implemented by e.g., a single circuit, unit or processor. Additionally, although individual features may be included in different claims, these may possibly be advantageously combined, and the inclusion in different claims does not imply that a combination of features is not feasible and/or advantageous. Also, the inclusion of a feature in one category of claims does not imply a limitation to this category but rather indicates that the feature is equally applicable to other claim categories as appropriate. Furthermore, the order of features in the claims do not imply any specific order in which the features must be worked and in particular the order of individual steps in a method claim does not imply that the steps must be performed in this order. Rather, the steps may be performed in any suitable order. In addition, singular references do not exclude a plurality. Thus, references to "a", "an", "first", "second" etc. do not preclude a plurality. Reference signs in the claims are provided merely as a clarifying example shall not be construed as limiting the scope of the claims in any way.

**Claims**

1. An apparatus comprising:

   a receiver (301) arranged to receive a data signal comprising compressed data for a biomedical waveform signal;
   a decompressor (303) arranged to generate a reconstructed biomedical waveform signal by decompression of the compressed data;
   wherein the data signal comprises an indication of a property of a compression process used to generate the compressed data from the biomedical waveform signal; and
   an adapter (307) arranged to adapt the decompression in dependence on the indication of the property of the compression.

2. The apparatus of claim 1 wherein the indication of the property of the compression process is indicative of a compression algorithm used to generate the compressed data; and the adapter (307) is arranged to select a decompression algorithm for the decompression from a set of decompression algorithms in dependence on the indication of the property of the compression process.

3. The apparatus of claim 2 wherein the set of decompression algorithms includes at least one lossless decompression algorithm and at least one lossy decompression algorithm.

4. The apparatus of any previous claim wherein the compressed data includes wavelet transform coefficient data, and the decompression includes applying a wavelet decompression algorithm to the wavelet transform coefficient data.

5. The apparatus of any of the previous claims wherein the indication of the property of the compression process is one of a plurality of indications of the property of the compression process each of which is linked with a time interval, and the adapter (307) is arranged to in each time interval adapt the decompression in dependence on the indication of the property of the compression linked with the time interval.

6. The apparatus of any previous claim wherein the compression employs a wavelet transform, and the indication of the property of the compression process comprises an indication of a mother wavelet for the wavelet transform.

7. The apparatus of any previous claim wherein the compression employs a wavelet transform, and the indication of the property of the compression process comprises an indication of a property of a composition tree for the wavelet transform.

8. The apparatus of any previous claim wherein the data signal comprises data for at least one further signal and a grouping indication for indicating that the biomedical waveform signal and the at least one further signal belong to a common group; and the decompressor (303) is arranged to generate the reconstructed biomedical waveform signal by generating the reconstructed biomedical waveform signal in dependence on the at least one further signal when the grouping indicator indicates that that the biomedical waveform signal and the at least one further signal belong to the common group.

9. The apparatus of claim 8 wherein the data signal further comprises a timing synchronization indicator indicative of a timing offset between the biomedical waveform signal and the at least one further signal.

10. The apparatus of any previous claim wherein the data signal comprises compressed data for a plurality of biomedical waveform signals and a type indicator indicating a biomedical waveform signal type for at least some of the biomedical waveform signals.

11. The apparatus of any previous claim wherein the data signal further comprises an indication of an average level of at least part of the biomedical waveform signal, and the decompression is arranged to generate the reconstructed biomedical waveform signal in dependence on the average level.

12. The apparatus of any previous claim wherein the compression property indication is indicative of segments of the biomedical waveform signal for which no data is included in the compressed data.

13. The apparatus of any previous claim wherein the data signal comprises at least one event indication for the biomedical waveform signal, the event indication being indicative of a timing of an event, and the adapter (307) is arranged to adapt the decompression in dependence on the event indication.

14. The apparatus of claim 1 wherein the data signal further comprises at least one indication of a first time interval, and the decompressor (303) is arranged to generate at least a component of the biomedical waveform signal for the first time interval independently of the compressed data.

15. An apparatus for generating a data signal representing a biomedical waveform signal, the apparatus comprising:

a receiver (201) arranged to receive the biomedical waveform signal;
a compressor (203) arranged to perform a compression process to generate compressed data from the biomedical waveform signal;
a data signal generator (205) arranged to generate the data signal to comprise the compressed data and an indication of a property of the compression process used to generate the compressed data from the biomedical waveform signal.

16. A data signal comprising:

compressed data for a biomedical waveform signal; and
an indication of a property of a compression process used to generate the compressed data from the biomedical waveform signal.

17. A method of reconstructing a biomedical waveform signal, the method comprising:

receiving a data signal comprising compressed data for the biomedical waveform signal;
generating a reconstructed biomedical waveform signal by decompression of the compressed data;
wherein the data signal comprises an indication of a property of a compression process used to generate the compressed data from the biomedical waveform signal; and the method comprises adapting the decompression in dependence on the indication of the property of the compression.

18. A method of generating a data signal representing a biomedical waveform signal, the method comprising:

receiving the biomedical waveform signal;
performing a compression process to generate compressed data from the biomedical waveform signal; and
generating the data signal to comprise the compressed data and an indication of a property of the compression process used to generate the compressed data from the biomedical waveform signal.

**FIG. 1**

**FIG. 2**

**FIG. 3**

FIG. 4

EP 4 732 757 A1

**FIG. 5**

Level coefficients

1

2

3

4

4

**FIG. 6**

**FIG. 7**

Processor 800

Core 802
- ALU 804
- FPLU 806
- DSPU 808

Registers 812

Cache 810

Controller 814

820A  820B  820C  820D

External Bus 816

ROM 832

Flash 834

RAM 833

EEPROM 835

Disc 836

**FIG. 8**

| | Europäisches Patentamt European Patent Office Office européen des brevets | **EUROPEAN SEARCH REPORT** | **Application Number** EP 24 20 8564 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRECHET L ET AL: "Compression of Biomedical Signals With Mother Wavelet Optimization and Best-Basis Wavelet Packet Selection", IEEE TRANSACTIONS ON BIOMEDICAL ENGINEERING, IEEE, USA, vol. 54, no. 12, 1 December 2007 (2007-12-01), pages 2186-2192, XP011195817, ISSN: 0018-9294, DOI: 10.1109/TBME.2007.896596 * the whole document *<br>----- | 1,2,4-7, 12,14-18 | INV. A61B5/00 H03M7/30 |
| X | BEKIRYAZICI TAHIR ET AL: "Electrocardiogram Signal Compression Using Deep Convolutional Autoencoder with Constant Error and Flexible Compression Rate", IRBM, ELSEVIER, AMSTERDAM, NL, vol. 45, no. 6, 6 September 2024 (2024-09-06), XP087651252, ISSN: 1959-0318, DOI: 10.1016/J.IRBM.2024.100859 [retrieved on 2024-09-06] * the whole document *<br>-----<br>-/-- | 1-3,5, 12,14-18 | TECHNICAL FIELDS SEARCHED (IPC)<br>A61B H03M |

The present search report has been drawn up for all claims

| Place of search | Date of completion of the search | Examiner |
|---|---|---|
| The Hague | 14 April 2025 | Scappazzoni, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PHAM NHAT ET AL: "PROS an efficient pattern-driven compressive sensing framework for low-power biopotential-based wearables with on-chip intelligence", PROCEEDINGS OF THE 6TH ANNUAL ACM SYMPOSIUM ON COMPUTATIONAL FABRICATION, ACMPUB27, NEW YORK, NY, USA, 14 October 2022 (2022-10-14), pages 661-675, XP058911880, DOI: 10.1145/3495243.3560533 ISBN: 978-1-4503-9262-4 * the whole document * | 1,2,5, 8-10, 12-18 | |
| X | US 2022/061771 A1 (QIN LUYUN [CN] ET AL) 3 March 2022 (2022-03-03) * paragraphs [0014] - [0023]; figures * | 1,2,5, 8-12, 14-18 | |
| | | | **TECHNICAL FIELDS SEARCHED (IPC)** |

The present search report has been drawn up for all claims

| Place of search | Date of completion of the search | Examiner |
|---|---|---|
| The Hague | 14 April 2025 | Scappazzoni, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

# EP 4 732 757 A1

## ANNEX TO THE EUROPEAN SEARCH REPORT
## ON EUROPEAN PATENT APPLICATION NO.

EP 24 20 8564

14-04-2025

| Patent document cited in search report | Publication date | Patent family member(s) | Publication date |
|---|---|---|---|
| US 2022061771 A1 | 03-03-2022 | CN 114098748 A | 01-03-2022 |
| | | US 2022061771 A1 | 03-03-2022 |

EPO FORM P0459